# EUROPÄISCHE PATENTSCHRIFT

(11) **EP 2 322 116 B1**
(45) Veröffentlichungstag und Bekanntmachung des Hinweises auf die Patenterteilung: **21.03.2012**
(21) Anmeldenummer: 09175751.8
(22) Anmeldetag: 12.11.2009
(51) Int. Cl.: A61C 19/00

(54) **Reinigungs- oder Pflegegerät für medizinische Instrumente**
Cleaning or maintaining device for medical instruments
Dispositif de nettoyage et d'entretien d'instruments médicaux

(43) Veröffentlichungstag der Anmeldung: 18.05.2011
(73) Patentinhaber: W & H Dentalwerk Bürmoos GmbH, 5111 Bürmoos (AT)
(72) Erfinder: Pfaffinger, Nikolaus, 5120, St. Pantaleon (AT); Spieler, Christian, 5152, Michaelbeuern (AT)
(74) Vertreter: Benda, Ralf

(56) Entgegenhaltungen:
- EP-A1- 1 749 502
- EP-A1- 1 949 869
- EP-A1- 1 982 667
- US-A- 5 415 248

## Beschreibung

Die vorliegende Erfindung betrifft ein Reinigungs- oder Pflegegerät zur Reinigung oder Pflege zumindest eines medizinischen, insbesondere dentalen, Instruments nach dem Oberbegriff des Anspruchs 1 sowie ein Verfahren zum Betrieb eines derartigen Reinigungs-oder Pflegegeräts.

Ein Reinigungs- oder Pflegegerät für medizinische, insbesondere dentale, Instrumente ist zum Beispiel aus dem Patent US 5,415,248 bekannt. Dieses Reinigungs-oder Pflegegerät weist einen Anschluss an eine Druckluftquelle auf und wird ausschließlich pneumatisch betrieben. Alle für die Reinigung oder Pflege notwendigen Funktionen, wie zum Beispiel die Förderung von Reinigungs- oder Pflegemitteln zu den Instrumenten oder der Antrieb mechanischer Bauteile der Instrumente während der Reinigung oder Pflege, werden somit direkt oder indirekt durch Druckluft bewirkt.

Der Vorteil dieses ausschließlich pneumatisch betriebenen Reinigungs- oder Pflegegeräts liegt in seinem einfachen Aufbau. Nachteilig ist jedoch, dass die Funktionalität des Reinigungs- oder Pflegegeräts beschränkt ist und nicht alle von den Anwendern gewünschte Funktionen realisierbar sind.

Aus der EP1749502 ist ein gattungsgemäßes Reinigungs- oder Pflegegerät bekannt.

Der vorliegenden Erfindung liegt daher die Aufgabe zugrunde, ein fluidbetriebenes Reinigungs- oder Pflegegerät für medizinische, insbesondere dentale, Instrumente sowie ein Verfahren zum Betrieb eines derartigen Reinigungs- oder Pflegegeräts zu schaffen, das diese Nachteile nicht aufweist und das dem Anwender bisher nicht realisierbare Funktionen bietet.

Gemäß einem Ausführungsbeispiel wird diese Aufgabe wird durch ein Reinigungs-oder Pflegegerät zum Durchführen zumindest einer Reinigungs- oder Pflegemaßnahme an zumindest einem medizinischen, insbesondere dentalen, Instrument gelöst, das einen Anschluss an eine Fluidquelle, insbesondere an eine Druckgasquelle, eine Versorgungsvorrichtung zur Versorgung des zumindest einen medizinischen, insbesondere dentalen, Instruments mit einem Reinigungs- oder Pflegemittel, einen durch eine bewegliche Türe oder eines beweglichen Deckel verschließbaren Reinigungs- oder Pflegeraum mit mehreren Kupplungen oder Anschlüssen für das zumindest eine medizinische, insbesondere dentale, Instrument, einen mit dem Anschluss an eine Fluidquelle verbundenen und durch ein Fluid betreibbaren elektrodynamischen Wandler zur Erzeugung elektrischer Energie und zumindest einen mit der von dem elektrodynamischen Wandler erzeugbaren elektrischen Energie versorgbaren elektrischen Verbraucher umfasst. Der elektrodynamische Wandler (im Folgenden auch als Generator bezeichnet) stellt dem Reinigungs- oder Pflegegerät in vorteilhafterweise elektrische Energie zur Verfügung, wodurch es möglich wird, in dem Reinigungs- oder Pflegegerät einen oder mehrere elektrische Verbraucher oder elektrisch betreibbare Bauteile vorzusehen, die die Funktionalität des Reinigungs- oder Pflegegeräts erweitern.

Eine besonders vorteilhafte Wirkung wird erzielt, wenn der elektrodynamische Wandler in einem Reinigungs- oder Pflegegerät vorgesehen ist, das keinen Anschluss an eine elektrische Energiequelle aufweist und das ausschließlich mit einem Fluid betrieben wird.

Als Reinigungs- oder Pflegegerät im Sinne des vorliegenden Schriftsatzes wird jede Vorrichtung bezeichnet, die zumindest ein Reinigungs- oder Pflegemittel an ein medizinisches, insbesondere dentales, Instrument abgibt, um dieses zu reinigen, zu desinfizieren, zu sterilisieren, von Verschmutzungen oder Mikroorganismen zu befreien oder zu pflegen. Als Reinigungs- oder Pflegemittel können zum Beispiel eine Flüssigkeit, insbesondere Heiß- oder Kaltwasser, Dampf, Desinfektions- oder Sterilisationsmittel, ein Gas, zum Beispiel Druckluft, oder ein Schmiermittel, zum Beispiel natürliche oder synthetische Schmieröle, verwendet werden. Die Reinigungs- oder Pflegemittel werden je nach Art des Instruments an dessen Außenseite oder in dessen Inneres, am gesamten Instrument oder nur an bestimmten Bauteilen des Instruments abgegeben.

Als Fluidquelle kann sowohl eine Flüssigkeitsquelle, insbesondere eine Wasserversorgung, oder eine Druckgasquelle, bevorzugt eine Druckluftquelle, vorgesehen sein.

Als zu reinigende medizinische, insbesondere dentale, Instrumente im Sinne des vorliegenden Schriftsatzes werden insbesondere Hand- und Winkelstücke zum Antrieb verschiedenster Werkzeuge, wie rotierender Bohrer, Zahnsteinentfernungswerkzeuge, Feilen, Sägen, Reamer usw., Handstücke zur Abgabe von medizinischen Materialien, wie zum Beispiel Füllmittel oder Anästhetika, Funktionshandstücke zur Abgabe von Licht, Wasser oder Luft und nicht angetriebene Handinstrumente, wie zum Beispiel medizinische Spiegel, Sonden, Trokare, die im Vorherstehenden genannten oder andere Werkzeuge, Endoskope und dergleichen verstanden.

Gemäß einem Ausführungsbeispiel ist der elektrodynamische Wandler als Wechselstromgenerator ausgebildet, der Wechselstrom oder Wechselspannung erzeugt und abgibt. Gemäß einem alternativen Ausführungsbeispiel ist der elektrodynamische Wandler als Gleichstromgenerator mit einer elektrischen oder mechanischen Kommutierungsvorrichtung ausgebildet, der Gleichstrom oder Gleichspannung abgibt.

Gemäß einem bevorzugten Ausführungsbeispiel umfasst der zumindest eine, mit der von dem elektrodynamischen Wandler erzeugten elektrischen Energie versorgbare elektrische Verbraucher zumindest eines der folgenden Elemente: Eine Fördervorrichtung, zum Beispiel eine Pumpe, eine elektrische oder elektronische Steuerung, einen elektrischen oder elektronischen Schaltkreis, einen Mikrocomputer, einen Aktor, ein Stellelement, ein Schaltelement, einen Sensor, einen Detektor, eine Beleuchtungsvorrichtung, zum Beispiel eine Leuchtdiode, ein Anzeigemittel, zum Beispiel ein Display oder zumindest eine Leuchtdiode, oder ein Antriebsmittel, zum Beispiel einen Elektromotor.

Gemäß einem anderen Ausführungsbeispiel weist das Reinigungs- oder Pflegegerät eine Speichereinheit zur Speicherung der von dem elektrodynamischen Wandler erzeugten elektrischen Energie auf. Als Speichereinheit werden zum Beispiel zumindest ein Kondensator oder zumindest ein wiederaufladbarer Akkumulator verwendet. Durch das Vorsehen der Speichereinheit können in vorteilhafter Weise der Betrieb des Generators und das Durchführen der zumindest einen Reinigungs- oder Pflegemaßnahme zeitlich voneinander getrennt werden. Dies ist insbesondere dann von Vorteil, wenn der dem Reinigungs- oder Pflegegerät zur Verfügung stehende Fluidstrom sowohl zum Betrieb des Generators als auch für die Reinigungs- oder Pflegemaßnahme eingesetzt wird und wenn der Volumenstrom oder der Betriebsdruck des Fluids nicht ausreichen, um den Betrieb des Generators und die zumindest eine Reinigungs- oder Pflegemaßnahme gleichzeitig durchzuführen. Dies trifft zum Beispiel auf ein besonders bevorzugtes Ausführungsbeispiel zu, bei dem als Fluid Druckluft verwendet wird, mit welcher der Generator betrieben wird und die zum Fördern oder Transport eines Reinigungs- oder Pflegemittels oder zum Durchblasen eines Instruments oder zum Trocknen eines Instruments genutzt wird. Demgemäß kann ein bevorzugtes Reinigungs- oder Pflegeverfahren zwei Verfahrensschritte aufweisen, wobei in einem Verfahrensschritt ausschließlich der Generator betrieben wird und die Speichereinheit aufgeladen wird und in einem weiteren Verfahrensschritt zumindest eine Reinigungs- oder Pflegemaßnahme durchgeführt wird, bei welcher bevorzugt in der Speichereinheit gespeicherte elektrische Energie verwendet wird oder einem elektrischen Verbraucher zugeführt wird. Besonders bevorzugt erfolgt der Verfahrensschritt, bei dem ausschließlich der Generator betrieben wird, unmittelbar vor dem Verfahrensschritt, der die Reinigungs-oder Pflegemaßnahme umfasst.

Bevorzugt weist das Reinigungs- oder Pflegegerät eine Steuervorrichtung auf, die ausgebildet ist, den elektrodynamischen Wandler zu aktivieren, um den elektrischen Energiegehalt der Speichereinheit zu erhöhen. Die Aktivierung des elektrodynamischen Wandlers erfolgt bevorzugt vor oder unmittelbar vor Beginn der Reinigungs- oder Pflegemaßnahme oder des gesamten Reinigungs- oder Pflegeverfahrens. Gemäß einer bevorzugten Weiterbildung umfasst das Reinigungs- oder Pflegegerät eine Steuervorrichtung, die ausgebildet ist, den elektrischen Energiegehalt der Speichereinheit zu bestimmen, insbesondere vor oder unmittelbar vor Beginn der Reinigungs- oder Pflegemaßnahme oder des gesamten Reinigungs- oder Pflegeverfahrens. Die Steuervorrichtung ist des Weiteren dazu ausgebildet, den elektrodynamischen Wandler zu aktivieren, um den elektrischen Energiegehalt der Speichereinheit zu erhöhen, insbesondere wenn der elektrische Energiegehalt der Speichereinheit zu gering ist, um die zumindest eine Reinigungs- oder Pflegemaßnahme oder das gesamte Reinigungs- oder Pflegeverfahren durchzuführen oder wenn der Energiegehalt der Speichereinheit unter einem vorbestimmten Grenzwert liegt. Damit wird in vorteilhafter Weise sichergestellt, dass der Generator nur betrieben wird, wenn die Speichereinheit nicht mehr ausreichend geladen oder leer ist und dass insbesondere zu Beginn der zumindest einen Reinigungs- oder Pflegemaßnahme oder des gesamten Reinigungs- oder Pflegeverfahrens ausreichend Energie in der Speichereinheit vorhanden ist, um die Reinigungs- oder Pflegemaßnahme oder das gesamten Reinigungs- oder Pflegeverfahrens ohne Unterbrechung durchführen zu können. Die Bestimmung des elektrischen Energiegehalts der Speichereinheit erfolgt zum Beispiel in bekannter Weise mittels einer Messung der elektrischen Spannung.

Gemäß einem Ausführungsbeispiel ist das Reinigungs- oder Pflegegerät derart ausgebildet, dass der elektrodynamische Wandler und der zumindest eine elektrische Verbraucher sequentiell betreibbar sind. Eine derartige Konstellation liegt zum Beispiel bei dem im Vorhinstehenden beschriebenen Reinigungs- oder Pflegeverfahren vor, bei dem der Betrieb des Generators und die zumindest eine Reinigungs- oder Pflegemaßnahme, während der auch der elektrische Verbraucher aktiv ist, hintereinander oder getrennt voneinander durchgeführt werden.

Gemäß einem alternativen Ausführungsbeispiel ist das Reinigungs- oder Pflegegerät derart ausgebildet, dass der elektrodynamische Wandler und der zumindest eine elektrische Verbraucher gleichzeitig betreibbar sind. Damit wird in vorteilhafter Weise die Betriebszeit des gesamten Reinigungs- oder Pflegeverfahrens verringert. Dieses Ausführungsbeispiel ist insbesondere dann realisierbar, wenn der Fluidstrom zum Betrieb des Generators nicht für die Reinigungs- oder Pflegemaßnahme eingesetzt wird.

Gemäß einem weiteren Ausführungsbeispiel umfasst das Reinigungs- oder Pflegegerät eine mit dem Anschluss an eine Fluidquelle verbundene Verteilereinheit, die ausgebildet ist, den vom Anschluss an eine Fluidquelle erhaltenen Fluidstrom alternierend entweder dem elektrodynamischen Wandler oder einem weiteren fluidleitenden oder fluidbetriebenen Element zuzuführen. Dieses Ausführungsbeispiel ist insbesondere von Vorteil, wenn der Betrieb des Generators und die zumindest eine Reinigungs- oder Pflegemaßnahme, während der auch das fluidleitende oder fluidbetriebene Element zum Einsatz kommt, hintereinander oder getrennt voneinander durchgeführt werden, so wie dies im Vorherstehenden bereits beschrieben wurde und wenn für den Generator und das fluidleitende oder fluidbetriebene Element nur ein einziger oder ein gemeinsamer Anschluss an eine Fluidquelle vorgesehen sind. Die Verteilereinheit ist bevorzugt als Ventil, insbesondere als Magnetventil ausgebildet. Besonders bevorzugt wird das Magnetventil mit elektrischer Energie des elektrodynamischen Wandlers betrieben.

Gemäß einer bevorzugten Weiterbildung dieses Ausführungsbeispiels umfasst die Verteilereinheit mehrere Ventile oder ist als Ventilblock mit mehreren Ventilen ausgebildet. Die Ventile steuern die alternierende Fluidzufuhr zum Generator und zu mehreren fluidleitenden oder fluidbetriebenen Elementen. Das weitere fluidleitende oder fluidbetriebene Element umfasst bevorzugt zumindest eine der folgenden Komponenten: Eine Fluidleitung, eine Fluidabgabevorrichtung, eine Düse, eine Fördervorrichtung, eine Pumpe, einen Aktor, ein Stellelement, ein Schaltelement, einen Sensor, einen Detektor oder ein Antriebsmittel.

Gemäß einem anderen Ausführungsbeispiel weist das Reinigungs- oder Pflegegerät einen elektrischen Gleichrichter zum Gleichrichten der vom elektrodynamischen Wandler, insbesondere der vom Wechselstromgenerator, erzeugten Wechselspannung auf. Damit kann der Generator in vorteilhafter Weise auch elektrische Verbraucher mit elektrischer Energie versorgen, die Gleichspannung oder Gleichstrom benötigen.

Ein Verfahren zum Betrieb eines in dem Schriftstück beschriebenen Reinigungs- oder Pflegegeräts umfasst den Schritt, dass der durch ein Fluid betreibbare elektrodynamische Wandler elektrische Energie erzeugt, um damit den zumindest einen elektrischen Verbraucher zu versorgen.

Gemäß einem Ausführungsbeispiel umfasst das Verfahren des Weiteren den Schritt, dass die Steuervorrichtung den elektrodynamischen Wandler aktiviert, um den elektrischen Energiegehalt der Speichereinheit zu erhöhen. Bevorzugt bestimmt die Steuervorrichtung den elektrischen Energiegehalt der Speichereinheit und aktiviert den elektrodynamischen Wandler, um den elektrischen Energiegehalt der Speichereinheit zu erhöhen, insbesondere wenn der elektrische Energiegehalt der Speichereinheit zu gering ist, um die zumindest eine Reinigungs- oder Pflegemaßnahme durchzuführen. Bevorzugt weist das Verfahren somit zwei Verfahrensschritte auf, wobei in einem Verfahrensschritt ausschließlich der Generator betrieben wird und die Speichereinheit aufgeladen wird und in einem weiteren Verfahrensschritt zumindest eine Reinigungs- oder Pflegemaßnahme durchgeführt wird, bei welcher bevorzugt in der Speichereinheit gespeicherte elektrische Energie verwendet wird oder einem elektrischen Verbraucher zugeführt wird. Besonders bevorzugt erfolgt der Verfahrensschritt, bei dem ausschließlich der Generator betrieben wird, unmittelbar vor dem Verfahrensschritt, der die Reinigungs- oder Pflegemaßnahme umfasst.

Gemäß einem anderen Ausführungsbeispiel weist das Verfahren den Schritt auf, dass der elektrodynamische Wandler und der zumindest eine elektrische Verbraucher sequentiell oder gleichzeitig betrieben werden.

Gemäß einem weiteren Ausführungsbeispiel weist das Verfahren den Schritt auf, dass die Verteilereinheit den vom Anschluss an eine Fluidquelle erhaltenen Fluidstrom entweder dem elektrodynamischen Wandler oder einem weiteren fluidleitenden oder fluidbetriebenen Element zuführt.

Die Erfindung wird nachfolgend anhand eines bevorzugten Ausführungsbeispiels und Bezug nehmend auf die beigefügten Zeichnungen erläutert:
Figur 1 zeigt in einer perspektivischen Ansicht ein Ausführungsbeispiel eines Reinigungs- oder Pflegegeräts.
Figur 2 zeigt in einer schematischen Darstellung den Aufbau des Reinigungs- oder Pflegegeräts der Figur 1.
Figur 3 zeigt ein Ausführungsbeispiel eines elektrodynamischen Wandlers zur Erzeugung elektrischer Energie.

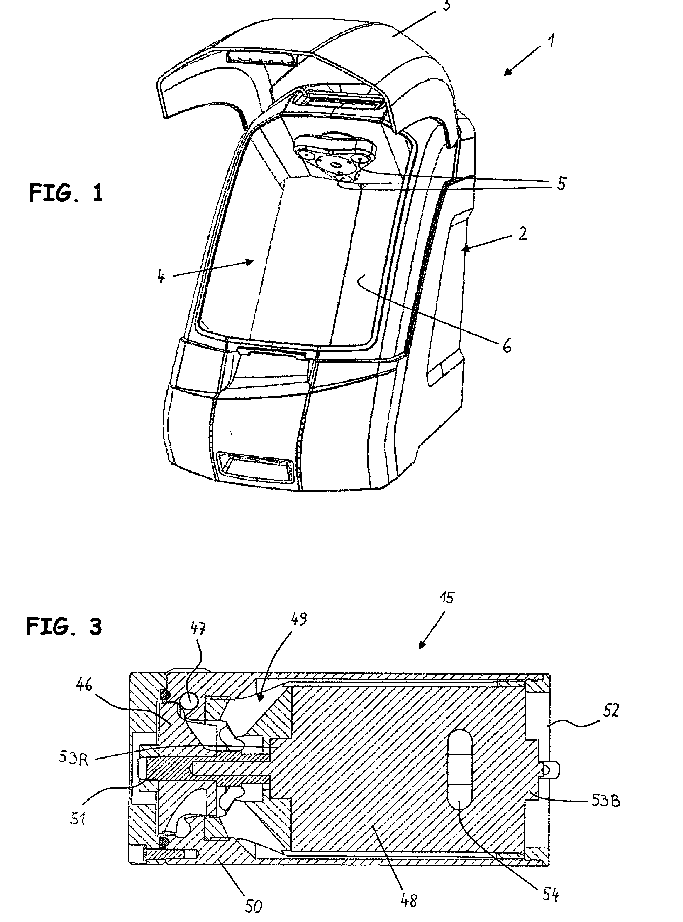

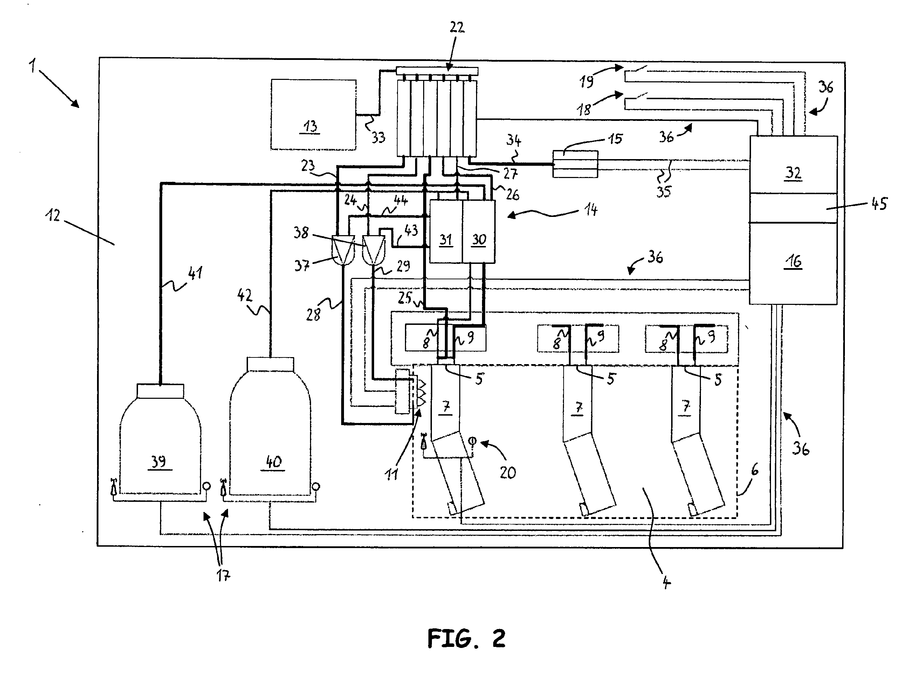

Das in Figur 1 dargestellte Reinigungs- oder Pflegegerät 1 umfasst ein, bevorzugt aus Kunststoff hergestelltes, Außengehäuse 2 mit einer Bodenplatte, vier Seitenwänden und einer Oberplatte. Eine der Seitenwände umfasst eine bewegliche, insbesondere verschwenkbare, Türe oder einen beweglichen, insbesondere verschwenkbaren, Deckel 3, der bevorzugt über Scharniere an der Oberplatte bewegbar ist. Mit dem Deckel 3 ist ein im Inneren des Gehäuses 2 angeordneter Reinigungs- oder Pflegeraum 4 verschließbar, in dem mehrere Kupplungen oder Anschlüsse 5, im vorliegenden Fall drei Anschlüsse, für zu reinigende oder zu pflegende medizinische, insbesondere dentale, Instrumente 7 (siehe Figur 2) vorgesehen sind. In den Anschlüssen 5 endet zumindest jeweils eine Medienleitung 8, 9, 25, über die ein Reinigungs- oder Pflegemittel in das Innere der Instrumente 7 förderbar ist. Zusätzlich befinden sich im Reinigungs- oder Pflegeraum 4 eine oder mehrere Öffnungen oder Düsen 11, über die ein Reinigungs- oder Pflegemittel auf die Außenseite der Instrumente 7 abgegeben werden kann.

Der Reinigungs- oder Pflegeraum 4 wird durch eine im Inneren des Reinigungs- oder Pflegegeräts 1 angeordnete Trennwand 6 von einem Steuerraum 12 (siehe Figur 2) getrennt. Der Steuerraum 12 enthält eine Vielzahl von im Folgenden näher beschriebenen Steuer- und Funktionselementen des Reinigungs- oder Pflegegeräts 1: Das Bezugszeichen 13 bezeichnet einen Anschluss an eine Fluidquelle, zum Beispiel an einen Kompressor, der das Reinigungs- oder Pflegegerät 1 mit Druckluft versorgt. Der Anschluss 13 ist bevorzugt lösbar mit einem Versorgungsschlauch verbunden, so dass das Reinigungs- oder Pflegegerät 1 von der Fluidquelle getrennt werden kann. Wie aus Figur 2 des Weiteren ersichtlich ist, ist der Anschluss 13 die einzige Verbindung des Reinigungs- oder Pflegegeräts 1 zu einer externen Energiequelle. Das Reinigungs- oder Pflegegerät 1 ist also ein nur durch ein Fluid (Druckluft) versorgtes Gerät, das insbesondere keinen Anschluss an eine elektrische Energiequelle aufweist.

Von dem Anschluss 13 führt eine Fluid-/Druckluftleitung 33 zu einer Verteilereinheit 22, die das Fluid / die Druckluft auf mehrere Bauelemente verteilt. Bevorzugt ist die Verteilereinheit 22 als Ventilblock ausgebildet, besonders bevorzugt als Ventilblock mit einem oder mehreren Magnetventilen, insbesondere als Ventilblock mit einem oder mehreren bistabilen Magnetventilen, die nur zum Umschalten zwischen der geöffneten Position und der geschlossenen Position einen Stromimpuls benötigen, nicht jedoch zum Aufrechterhalten der jeweiligen Position. Von der Verteilereinheit 22 führen mehrere Fluid/Druckluftleitungen 23, 24, 25, 26, 27 zu einer Versorgungsvorrichtung 14 zur Versorgung der Instrumente 7 mit einem Reinigungs- oder Pflegemittel sowie eine weitere Fluid-/Druckluftleitung 34 zu einem elektrodynamischen Wandler 15 oder Generator zur Erzeugung elektrischer Energie.

Der Generator 15 wird durch das Fluid / die Druckluft angetrieben und erzeugt eine elektrische Wechselspannung, die über zwei elektrische Leitungen 35 einem elektrischen Gleichrichter 32 zum Gleichrichten zugeführt wird. Mit dem Gleichrichter 32 ist eine Speichereinheit 45 zur Speicherung der von dem elektrodynamischen Wandler 15 erzeugten elektrischen Energie verbunden. Die Speichereinheit 45 umfasst bevorzugt zumindest einen Kondensator. Die vom Generator 15 erzeugte elektrische Energie dient zur Versorgung und zum Betrieb verschiedener elektrischen Verbraucher, insbesondere von im Weiteren noch im Detail beschriebenen Sensoren 17, 18, 19, 20, des Magnetventilblocks 22 und / oder einer Steuervorrichtung oder einem Microcomputer 16. Die Verbindung zwischen dem Generator 15 bzw. der Speichereinheit 45 und den elektrischen Verbrauchern 16 - 20, 22 erfolgt über elektrische Leitungen 36.

Die Steuervorrichtung 16 dient der Steuerung und Regelung des Reinigungs- oder Pflegegeräts 1 und dessen Funktionselementen sowie der Steuerung und Regelung der von dem Reinigungs- oder Pflegegerät 1 durchführbaren Verfahren, insbesondere der Reinigungs- und Pflegemaßnahmen sowie der Erzeugung der elektrischen Energie. Die Steuervorrichtung 16 speichert zum Beispiel unterschiedliche Reinigungs- oder Pflegeprogramme für unterschiedliche Instrumente 7, steuert oder regelt die Versorgungsvorrichtung 14 zur Versorgung der Instrumente 7 mit den Reinigungs- oder Pflegemitteln, so dass die unterschiedlichen Instrumente 7 nur mit den für sie geeigneten Reinigungs- und Pflegemaßnahmen behandelt werden, empfängt von Stellelementen, zum Beispiel der Verteilereinheit 22, oder den Sensoren 17, 18, 19, 20 Signale oder sendet über elektrische Leitungen 36 Signale an die Stellelemente 22 oder die Sensoren 17, 18, 19, 20, verarbeitet die empfangenen Signale und steuert Anzeigevorrichtungen, zum Beispiel Leuchtdioden, insbesondere zum Anzeigen von Betriebszuständen oder Fehlfunktionen.

Die Steuervorrichtung 16 ist auch dazu ausgebildet, den elektrischen Energiegehalt der Speichereinheit 45 zu bestimmen, zum Beispiel mittels einer Spannungsmessung, und den elektrodynamischen Wandler 15 durch Öffnen des entsprechenden Ventils der Verteilereinheit 22 zu aktivieren, um den elektrischen Energiegehalt der Speichereinheit 45 zu erhöhen, insbesondere wenn der bestimmte elektrische Energiegehalt der Speichereinheit 45 zu gering ist, um die zumindest eine Reinigungs- oder Pflegemaßnahme durchzuführen. Alternativ ist die Steuervorrichtung 16 ausgebildet, den Generator 15 vor Beginn der Reinigungs- und Pflegemaßnahmen zu aktivieren, ohne dessen Energiegehalt zu bestimmen.

Zwei Leitungen 23, 24 erstrecken sich von der Verteilereinheit 22 bzw. von jeweils einem Ventil der Verteilereinheit 22 zu zwei Mischeinheiten 37, 38. In diesen Mischeinheiten 37, 38 werden ein oder mehrere Reinigungs- oder Pflegemittel mit der von den Leitungen 23, 24 zugeführten Druckluft vermischt oder in den Druckluftstrom eingebracht, so dass ein Reinigungs- oder Pflegemittel-Luft-Gemisch entsteht, das über die Leitungen 28, 29 einer Abgabevorrichtung zugeführt und über die Düsen 11 der Abgabevorrichtung auf die Außenseite der Instrumente 7 abgegeben wird.

Eine Leitung 25 erstreckt sich von der Verteilereinheit 22 bzw. von einem Ventil der Verteilereinheit 22 zu dem Anschluss 5, so dass das Fluid, insbesondere die Druckluft, durch das Innere des Instruments 7 oder durch Bauteile des Instruments 7, zum Beispiel durch Leitungen oder Rohre, gefördert werden kann.

Zwei weitere Leitungen 26, 27 verbinden die Verteilereinheit 22 bzw. jeweils ein Ventil der Verteilereinheit 22 mit jeweils einer Pumpe 30, 31. Die Pumpen 30, 31 sind bevorzugt als Kolbenpumpen ausgebildet, die durch das Fluid, insbesondere die Druckluft, betrieben werden, um ein oder mehrere Reinigungs- oder Pflegemittel aus den Vorratsbehältern 39, 40 zu fördern. Die Pumpen 30, 31 sind über Leitungen 41, 42 mit den Vorratsbehältern 39, 40 verbunden. Über zwei weitere Leitungen 8, 9 wird das Reinigungs- oder Pflegemittel dem Anschluss 5 zugeführt, von wo es in das Innere des Instruments 7 oder in Bauteile des Instruments 7 gelangt. Schließlich verbinden zwei Leitungen 43, 44 die Pumpen 30, 31 mit den Mischeinheiten 37, 38, so dass, wie im Vorherstehenden schon beschrieben, in den Mischeinheiten 37, 38 Reinigungs- oder Pflegemittel mit der Druckluft vermischt wird. Wie ebenfalls im Vorherstehenden bereits erwähnt, steuert die Steuervorrichtung 16 die Pumpen 30, 31 und / oder die Versorgungsvorrichtung 14, insbesondere den Fluidfluss durch deren Leitungen 23 - 27, und somit die Art und / oder die Abfolge der auf die Instrumente 7 abgegebenen Reinigungs- oder Pflegemittel. Die Steuerung der Pumpen 30, 31 und / oder der Versorgungsvorrichtung 14 erfolgt dabei durch Öffnen oder Schließen der Ventile der Verteilereinheit 22.

Jedem Vorratsbehälter 39, 40 ist ein Sensor 17 zur Bestimmung des Füllstands zugeordnet. Der Füllstandsensor 17 ist bevorzugt als Infrarot-Sensor ausgebildet, er kann jedoch selbstverständlich auch andere bekannte Sensortypen umfassen. Ist der Füllstand eines der Vorratsbehälter 39, 40 gering oder ist einer der Vorratsbehälter 39, 40 leer, so sendet der jeweilige Füllstandsensor 17 über die elektrische Leitung 36 ein Signal an die Steuervorrichtung 16, die als Reaktion auf dieses Signal eine an der Außenseite des Reinigungs- oder Pflegegeräts 1 angeordnete Anzeigevorrichtung, zum Beispiel eine Leuchtdiode, aktiviert, die den Anwender über den geringen Füllstand informiert.

Am Deckel 3 oder an der Kontaktstelle zwischen dem Deckel 3 und dem Außengehäuse 2 ist ein Sensor 18 vorgesehen, der detektiert, ob der Deckel 3 am Außengehäuse 2 anliegt oder ob er nicht anliegt, i.e. ob der Reinigungs- oder Pflegeraum 4 durch den Deckel 3 verschlossen ist oder nicht. Der Sensor 18 ist bevorzugt als Drucksensor oder Mikroschalter ausgebildet, er kann jedoch selbstverständlich auch andere bekannte Sensortypen umfassen. Ist der Reinigungs- oder Pflegeraum 4 durch den Deckel 3 nicht verschlossen, so empfängt die Steuervorrichtung 16 vom Sensor 18 über die elektrische Leitung 36 ein Signal und aktiviert als Reaktion auf dieses Signal eine an der Außenseite des Reinigungs- oder Pflegegeräts 1 angeordnete Anzeigevorrichtung, zum Beispiel eine Leuchtdiode.

Zum Auffangen der über die Düsen 11 und / oder über die Anschlüsse 5 in den Reinigungs- oder Pflegeraum 4 abgegebenen Reinigungs- oder Pflegemittel ist im Bereich der Bodenplatte des Reinigungs- oder Pflegegeräts 1 eine Auffangwanne vorgesehen. An der Auffangwanne ist ein Füllstandsensor 19 vorgesehen, der ein Überlaufen der Auffangwanne verhindert. Der Füllstandsensor 19 umfasst bevorzugt einen Schwimmer, insbesondere einen Schwimmer mit einem Magneten, und einen Magnetsensor, zum Beispiel einen Hall- oder Reed-Sensor, er kann jedoch selbstverständlich auch andere bekannte Sensortypen umfassen. Erreicht der Füllstand in der Auffangwanne einen vorbestimmten Wert, so sendet der Füllstandsensor 19 über die elektrische Leitung 36 ein Signal an die Steuervorrichtung 16, die als Reaktion auf dieses Signal eine an der Außenseite des Reinigungs- oder Pflegegeräts 1 angeordnete Anzeigevorrichtung, zum Beispiel eine Leuchtdiode, aktiviert, die den Anwender über den Füllstand informiert.

Ein weiterer Sensor 20 ist im Reinigungs- oder Pflegeraum 4 angeordnet und dient zur Erkennung, ob an die Anschlüsse 5 ein Instrument 7 angeschlossen ist oder nicht oder welche Art von Instrument 7 an den jeweiligen Anschluss angeschlossen ist. Der Sensor 20 ist bevorzugt als Infrarot-Sensor ausgebildet, er kann jedoch selbstverständlich auch andere bekannte Sensortypen umfassen. Die Steuervorrichtung 16 erhält vom Sensor 20 über die elektrische Leitung 36 ein Signal, auf dessen Basis sie bestimmt, ob auf den jeweiligen Anschlüssen 5 ein Instrument 7 angeschlossen ist oder nicht oder welche Art von Instrument 7 an den jeweiligen Anschluss angeschlossen ist und auf dessen Basis sie dem jeweiligen Anschluss 5 kein oder ein oder mehrere Reinigungs- oder Pflegemittel zukommen lässt, in dem sie die Verteilereinheit 22 entsprechend ansteuert, so wie dies im Vorherstehenden bereits beschrieben wurde.

Die zum Betrieb und zur Erzeugung der Signale benötigte elektrische Energie erhalten die Sensoren 17, 18, 19, 20 vom Generator 15 oder von der elektrischen Speichereinheit 45.

Ein bevorzugtes Verfahren zum Betrieb des Reinigungs- oder Pflegegeräts 1 weist zwei Verfahrensschritte auf, wobei in einem Verfahrensschritt ausschließlich der Generator 15 betrieben wird, um die Speichereinheit 45 aufzuladen, und in einem weiteren Verfahrensschritt zumindest eine Reinigungs- oder Pflegemaßnahme durchgeführt wird. Bevorzugt ist die Steuervorrichtung 16 derart ausgebildet, dass sie den Betrieb des Generators 15 und die zumindest eine Reinigungs- oder Pflegemaßnahme sequentiell durchführt, wobei sie insbesondere den Generator 15 vor oder unmittelbar vor der Reinigungs- oder Pflegemaßnahme aktiviert.

Der Aufbau des Generators 15 ist in Figur 3 dargestellt: Er umfasst ein Laufrad 46, eine Fluidzuführung 47, der, gegebenenfalls über Leitelemente, den Fluidstrom auf das Laufrad 46 lenkt, ein Rotor-Stator-Paket 48, eine Fluidabführung 49 und eine ein- oder mehrteilige Außenhülse 50. Das Laufrad 46 ist auf einer ein- oder mehrteiligen Wellenanordnung 51 befestigt und über diese Wellenanordnung 51 mit dem Rotor des Rotor-Stator-Pakets 48 verbunden. Der Rotor umfasst ein Magnetelement, das drehbar innerhalb des Stators gelagert ist. Der Stator umfasst mehrere Spulen oder Wicklungen, ein Magnetflusselement und elektrische Kontakte oder Leitungen zur elektrische Verbindung des Generators 15 mit den elektrischen Verbrauchern 16 - 20, 22.

Die Fluidzuführung 47 umfasst eine die Außenhülse 50 des Generators 15 durchsetzende Bohrung oder einen Kanal. Das über die Fluidzuführung 47 zugeführte Fluid setzt das Laufrad 46 in Rotation, wobei die Rotationsbewegung über die Wellenanordnung 51 auf den Rotor des Rotor-Stator-Pakets 48 übertragen wird, so dass in bekannter Weise eine elektrische Wechselspannung generiert wird. Mehrere Wälzlager 53A, 53B lagern die Wellenanordnung 51 und den Rotor des Rotor-Stator-Pakets 48.

Die Fluidabführung 49 erstreckt sich durch Kanäle oder Bohrungen innerhalb der Hülse 50 vom Laufrad 46 entlang des Außenumfangs des Rotor-Stator-Pakets 48, durch Bohrungen 54 im Rotor-Stator-Paket 48 und durch das Innere des Rotor-Stator-Pakets 48 bis zu einer Luftauslassöffnung 52 in der Hülse 50. Von dort wird das Fluid durch eine oder mehrere Öffnungen im Außengehäuse 2 des Reinigungs- oder Pflegegeräts 1 an die Umgebung abgegeben.

Die Erfindung ist nicht auf das beschriebene Ausführungsbeispiel beschränkt, sondern umfasst alle Ausführungen, die in den Schutzumfang der Erfindung fallen, der in den angegebenen Ansprüchen dargelegt ist. Des Weiteren sind alle Merkmale aller beschriebenen und dargestellten Ausführungsbeispiele miteinander kombinierbar.

## Patentansprüche

1. Reinigungs- oder Pflegegerät (1) zum Durchführen zumindest einer Reinigungs- oder Pflegemaßnahme an zumindest einem medizinischen, insbesondere dentalen, Instrument (7), umfassend einen Anschluss (13) an eine Fluidquelle, insbesondere an eine Druckgasquelle, eine Versorgungsvorrichtung (14) zur Versorgung des zumindest einen medizinischen, insbesondere dentalen, Instruments (7) mit einem Reinigungs- oder Pflegemittel, und einen durch eine bewegliche Türe oder einen beweglichen Deckel (3) verschließbaren Reinigungs- oder Pflegeraum (4) mit mehreren Kupplungen oder Anschlüssen (5) zum Anschluss des zumindest einen zu reinigenden oder zu pflegenden medizinischen, insbesondere dentalen, Instruments (7), **gekennzeichnet durch**
einen mit dem Anschluss (13) an eine Fluidquelle verbundenen und **durch** ein Fluid betreibbaren elektrodynamischen Wandler (15) zur Erzeugung elektrischer Energie und **durch** zumindest einen mit der von dem elektrodynamischen Wandler (15) erzeugbaren elektrischen Energie versorgbaren elektrischen Verbraucher (16 - 20, 22).

2. Reinigungs- oder Pflegegerät (1) nach Anspruch 1, **gekennzeichnet durch** eine Speichereinheit (45) zur Speicherung der von dem elektrodynamischen Wandler (15) erzeugten elektrischen Energie.

3. Reinigungs- oder Pflegegerät (1) nach Anspruch 1 oder 2, **gekennzeichnet durch** eine Steuervorrichtung (16), die ausgebildet ist, den elektrodynamischen Wandler (15) zu aktivieren.

4. Reinigungs- oder Pflegegerät (1) nach einem der vorhergehenden Ansprüche, **dadurch gekennzeichnet, dass**
der elektrodynamische Wandler (15) und der zumindest eine elektrische Verbraucher (16 - 20, 22) sequentiell oder gleichzeitig betreibbar sind.

5. Reinigungs- oder Pflegegerät (1) nach einem der vorhergehenden Ansprüche, **dadurch gekennzeichnet, dass**
der zumindest eine, mit der von dem elektrodynamischen Wandler (15) erzeugbaren elektrischen Energie versorgbare elektrische Verbraucher (16 - 20, 22) zumindest eines der folgenden Elemente umfasst: Eine Fördervorrichtung, eine Pumpe, eine elektrische oder elektronische Steuerung (16), einen elektrischen oder elektronischen Schaltkreis, einen Mikrocomputer, einen Aktor, ein Stellelement, ein Schaltelement, einen Sensor (17 - 20), einen Detektor, eine Beleuchtungsvorrichtung, ein Anzeigemittel oder ein Antriebsmittel.

6. Reinigungs- oder Pflegegerät (1) nach einem der vorhergehenden Ansprüche, **gekennzeichnet durch**
eine mit dem Anschluss (13) an eine Fluidquelle verbundene Verteilereinheit (22), die ausgebildet ist, den vom Anschluss (13) an eine Fluidquelle erhaltenen Fluidstrom entweder dem elektrodynamischen Wandler (15) oder einem weiteren fluidleitenden oder fluidbetriebenen Element (11; 23-29; 30, 31) zuzuführen.

7. Reinigungs- oder Pflegegerät (1) nach Anspruch 6, **dadurch gekennzeichnet, dass** das weitere fluidleitende oder fluidbetriebene Element (11; 23-29; 30, 31) zumindest eine der folgenden Komponenten umfasst: Eine Fluidleitung (23 - 29), eine Fluidabgabevorrichtung, eine Düse (11), eine Fördervorrichtung, eine Pumpe (30, 31), einen Aktor, ein Stellelement, ein Schaltelement, einen Sensor, einen Detektor oder ein Antriebsmittel.

8. Reinigungs- oder Pflegegerät (1) nach einem der vorhergehenden Ansprüche, **gekennzeichnet durch**
einen elektrischen Gleichrichter (32) zum Gleichrichten der vom elektrodynamischen Wandler (15) erzeugten Wechselspannung.

9. Verfahren zum Betrieb eines Reinigungs- oder Pflegegeräts (1) nach einem der vorhergehenden Ansprüche, **dadurch gekennzeichnet, dass**
der durch ein Fluid betreibbare elektrodynamische Wandler (15) elektrische Energie erzeugt, um damit den zumindest einen elektrischen Verbraucher (16 - 20, 22) zu versorgen.

10. Verfahren zum Betrieb eines Reinigungs- oder Pflegegeräts (1) nach Anspruch 9, **dadurch gekennzeichnet, dass**
die Steuervorrichtung (16) den elektrodynamischen Wandler (15) aktiviert, damit dieser elektrische Energie erzeugt.

11. Verfahren zum Betrieb eines Reinigungs- oder Pflegegeräts (1) nach Anspruch 9 oder 10, **dadurch gekennzeichnet, dass**
der elektrodynamische Wandler (15) und der zumindest eine elektrische Verbraucher (16 - 20, 22) sequentiell oder gleichzeitig betrieben werden.

12. Verfahren zum Betrieb eines Reinigungs- oder Pflegegeräts (1) nach einem der Ansprüche 9 - 11, **dadurch gekennzeichnet, dass**
die Verteilereinheit (22) den vom Anschluss (13) an eine Fluidquelle erhaltenen Fluidstrom entweder dem elektrodynamischen Wandler (15) oder einem weiteren fluidleitenden oder fluidbetriebenen Element (11; 23-29; 30, 31) zuführt.

## Claims

1. A cleaning or care device (1) for performing at least one cleaning or care measure on at least one medical, in particular dental, instrument (7), comprising a connection (13) to a fluid source, in particular to a compressed gas source, a supply device (14) for supplying the at least one medical, in particular dental, instrument (7) with a cleaning or care agent, and a cleaning or care space (4) with multiple couplings or connections (5) for connecting the at least one medical, in particular dental, instrument (7) that is to be cleaned or cared for, and which can be closed by a movable door or a movable cover (3), **characterized by**
an electrodynamic transducer (15) which is connected to the connection (13) to a fluid source and can be operated by a fluid for generating electric power and by at least one electric consumer (16 - 20, 22) which can be supplied with electric power that can be generated by the electrodynamic transducer (15).

2. The cleaning or care device (1) according to Claim 1, **characterized by** a storage unit (45) for storing the electric power generated by the electrodynamic transducer (15).

3. The cleaning or care device (1) according to Claim 1 or 2, **characterized by** a control device (16) which is designed to activate the electrodynamic transducer (15).

4. The cleaning or care device (1) according to any one of the preceding claims, **characterized in that**
the electrodynamic transducer (15) and the at least one electric consumer (16 - 20, 22) can be operated sequentially or simultaneously.

5. The cleaning or care device (1) according to any one of the preceding claims, **characterized in that**
the at least one electric consumer (16-20, 22) which can be supplied with electric power that can be generated by the electrodynamic transducer (15) comprises at least one of the following elements: a delivery device, a pump, an electric or electronic control device (16), an electric or electronic circuit, a microcomputer, an actuator, an actuating element, a switch element, a sensor (17 - 20), a detector, an illumination device, a display means or a drive means.

6. The cleaning or care device (1) according to any one of the preceding claims, **characterized by**
a distributor unit (22) which is connected to the connection (13) to a fluid source and is designed to supply the fluid stream obtained from the connection (13) to a fluid source to either the electrodynamic transducer (15) or to another fluid-conducting or fluid-operated element (11; 23 - 29; 30, 31).

7. The cleaning or care device (1) according to Claim 6, **characterized in that** the another fluid-conducting or fluid-operated element (11; 23 - 29; 30, 31) comprises at least one of the following components: a fluid line (23 - 29), a fluid-dispensing device, a nozzle (11), a delivery device, a pump (30, 31), an actuator, an actuating element, a switch element, a sensor, a detector or a drive means.

8. The cleaning or care device (1) according to any one of the preceding claims, **characterized by**
an electric rectifier (32) for rectifying the alternating voltage generated by the electrodynamic transducer (15).

9. A method for operating a cleaning or care device (1) according to any one of the preceding claims, **characterized in that**
the electrodynamic transducer (15) operable by a fluid generates electric power to thereby supply power to the at least one electric consumer (16 - 20, 22).

10. The method for operating a cleaning or care device (1) according to Claim 9, **characterized in that**
the control device (16) activates the electrodynamic transducer (15), so that the latter will produce electric power.

11. The method for operating a cleaning or care device (1) according to Claim 9 or 10, **characterized in that**
the electrodynamic transducer (15) and the at least one electric consumer (16 - 20, 22) are operated sequentially or simultaneously.

12. The method for operating a cleaning or care device (1) according to any one of Claims 9 - 11, **characterized in that**
the distributor unit (22) supplies the fluid stream obtained from the connection (13) to a fluid source to either the electrodynamic transducer (15) or to another fluid-conducting or fluid-operated element (11; 23 - 29; 30, 31).

## Revendications

1. Appareil de nettoyage ou d'entretien (1) pour effectuer au moins une mesure de nettoyage ou d'entretien sur au moins un instrument (7) médical, en particulier dentaire, comprenant un raccord (13) à une source de fluide, en particulier à une source de gaz sous pression, un dispositif d'alimentation (14) pour fournir un agent de nettoyage ou d'entretien à l'au moins un instrument (7) médical, en particulier dentaire, et un espace de nettoyage ou d'entretien (4) pouvant être fermé par une porte mobile ou un couvercle mobile (3) avec plusieurs accouplements ou raccords (5) pour raccorder l'au moins un instrument (7) médical, en particulier dentaire, à nettoyer ou à entretenir, **caractérisé par**
un convertisseur électrodynamique (15) relié au raccord (13) à une source de fluide (13) et pouvant être entraîné par un fluide pour produire de l'énergie électrique, et par au moins un récepteur électrique (16 - 20, 22) pouvant être alimenté avec l'énergie électrique pouvant être produite par le convertisseur électrodynamique (15).

2. Appareil de nettoyage ou d'entretien (1) selon la revendication 1, **caractérisé par** une unité de stockage (45) pour stocker l'énergie électrique produite par le convertisseur électrodynamique (15).

3. Appareil de nettoyage ou d'entretien (1) selon la revendication 1 ou 2, **caractérisé par** un dispositif de commande (16) qui est formé pour activer le convertisseur électrodynamique (15).

4. Appareil de nettoyage ou d'entretien (1) selon l'une des revendications précédentes, **caractérisé en ce que**
le convertisseur électrodynamique (15) et l'au moins un récepteur électrique (16 - 20, 22) peuvent être entraînés de manière séquentielle ou simultanée.

5. Appareil de nettoyage ou d'entretien (1) selon l'une des revendications précédentes, **caractérisé en ce que**
l'au moins un récepteur électrique (16 - 20, 22) pouvant être alimenté avec l'énergie électrique pouvant être produite par le convertisseur électrodynamique (15), comprend au moins un des éléments suivants : un dispositif de transport, une pompe, une commande électrique ou électronique (16), un circuit de commutation électrique ou électronique, un micro-ordinateur, un actionneur, un élément de réglage, un élément de commutation, un capteur (17 - 20), un détecteur, un dispositif d'éclairage, un dispositif d'affichage ou un dispositif d'entraînement.

6. Appareil de nettoyage ou d'entretien (1) selon l'une des revendications précédentes, **caractérisé par**
une unité distributrice (22) reliée au raccord (13) à une source de fluide, laquelle est formée pour amener l'écoulement de fluide obtenu par le raccord (13) à une source
de fluide, soit au convertisseur électrodynamique (15), soit à un autre élément conduisant le fluide ou entraîné par le fluide (11; 23 - 29; 30, 31).

7. Appareil de nettoyage ou d'entretien (1) selon la revendication 6, **caractérisé en ce que**
l'autre élément conduisant le fluide ou entraîné par le fluide (11; 23 - 29; 30, 31) comprend au moins un des composants suivants: une conduite de fluide (23 - 29), un dispositif de distribution de fluide, un injecteur (11), un dispositif de transport, une pompe (30, 31), un actionneur, un élément de réglage, un élément de commutation, un capteur, un détecteur ou un moyen d'entraînement.

8. Appareil de nettoyage ou d'entretien (1) selon l'une des revendications précédentes, **caractérisé par**
un redresseur électrique (32) pour redresser la tension alternative produite par le convertisseur électrodynamique (15).

9. Procédé d'actionnement d'un appareil de nettoyage ou d'entretien (1) selon l'une des revendications précédentes, **caractérisé en ce que**
e convertisseur électrodynamique (15) pouvant être actionné par un fluide produit de l'énergie électrique pour alimenter l'au moins un récepteur électrique (16 - 20, 22) avec celle-ci.

10. Procédé d'actionnement d'un appareil de nettoyage ou d'entretien (1) selon la revendication 9, **caractérisé en ce que**
le dispositif de commande (16) active le convertisseur électrodynamique (15) afin que celui-ci produise de l'énergie.

11. Procédé d'actionnement d'un appareil de nettoyage ou d'entretien (1) selon la revendication 9 ou 10, **caractérisé en ce que**
le convertisseur électrodynamique (15) et l'au moins un récepteur électrique (16 - 20, 22) sont entraînés de manière séquentielle ou simultanée.

12. Procédé d'actionnement d'un appareil de nettoyage ou d'entretien (1) selon l'une des revendication 9 à 11, **caractérisé en ce que**
l'unité distributrice (22) amène l'écoulement de fluide obtenu par le raccord (13) à une source de fluide, soit au convertisseur électrodynamique (15), soit à un autre élément conduisant le fluide ou entraîné par le fluide (11; 23-29; 30, 31).
